# EUROPEAN PATENT APPLICATION

(11) **EP 2 213 288 A1**
(43) Date of publication of application: **04.08.2010**
(21) Application number: 09001287.3
(22) Date of filing: 30.01.2009
(51) Int. Cl.: A61K 31/455

(54) **NAMPT and vitamin B3 for treating or preventing diseases**

(71) Applicant: Welte, Karl, 30657 Hannover (DE); Skokowa, Julia, 30625 Hannover (DE)
(72) Inventor: Welte, Karl, 30657 Hannover (DE); Skokowa, Julia, 30625 Hannover (DE)
(74) Representative: Kröncke, Rolf

(57) **Abstract**

The present invention relates to the use of a vitamin B3 and compounds derived therefrom as well as to nicotinamide phosphoribosyltransferase (NAMPT) and derivatives thereof as well as enhancers or inducers of NAMPT expression, NAMPT activity or NAMPT-mediated signalling for preventing or treating all types of cytopenia of the myeloid or lymphoid lineage. In a particular embodiment, the present invention relates to the use of the above referenced compounds for treating neutropenia, like severe congenital neutropenia or chemotherapy-induced febrile neutropenia or other congenital or acquired neutropenia. The invention also relates to the mobilisation of hematopoietic stem cells into peripheral blood. In another aspect, the present invention relates to pharmaceutical compositions containing as active ingredients vitamin B3 and metabolites thereof or NAMPT and modulators of the NAMPT activity, NAMPT expression or NAMPT mediated signalling in a combination with a therapeutically active or suboptimal amount of G-CSF.

SEQUENCE LISTING

<110> Welte, Karl
      Skokowa, Julia

<120> NAMPT and vitaminB3 for treating or preventing diseases

<130> 4624-001

<160> 3

<170> PatentIn version 3.3

<210> 1
<211> 1476
<212> DNA
<213> human

<220>
<221> CDS
<222> (1)..(1476)

<400> 1
atg aat cct gcg gca gaa gcc gag ttc aac atc ctc ctg gcc acc gac      48
tcc tac aag gtt act cac tat aaa caa tat cca ccc aac aca agc aaa      96
gtt tat tcc tac ttt gaa tgc cgt gaa aag aag aca gaa aac tcc aaa      144
tta agg aag gtg aaa tat gag gaa aca gta ttt tat ggg ttg cag tac      192
att ctt aat aag tac tta aaa ggt aaa gta gta acc aaa gag aaa atc      240
cag gaa gcc aaa gat gtc tac aaa gaa cat ttc caa gat gat gtc ttt      288
aat gaa aag gga tgg aac tac att ctt gag aag tat gat ggg cat ctt      336
cca ata gaa ata aaa gct gtt cct gag ggc ttt gtc att ccc aga gga      384
aat gtt ctc ttc acg gtg gaa aac aca gat cca gag tgt tac tgg ctt      432
aca aat tgg att gag act att ctt gtt cag tcc tgg tat cca atc aca      480
gtg gcc aca aat tct aga gag cag aag aaa ata ttg gcc aaa tat ttg      528
tta gaa act tct ggt aac tta gat ggt ctg gaa tac aag tta cat gat     576
ttt ggc tac aga gga gtc tct tcc caa gag act gct ggc ata gga gca     624
tct gct cac ttg gtt aac ttc aaa gga aca gat aca gta gca gga ctt     672
gct cta att aaa aaa tat tat gga acg aaa gat cct gtt cca ggc tat     720
tct gtt cca gca gca gaa cac agt acc ata aca gct tgg ggg aaa gac     768
cat gaa aaa gat gct ttt gaa cat att gta aca cag ttt tca tca gtg     816
cct gta tct gtg gtc agc gat agc tat gac att tat aat gcg tgt gag     864
aaa ata tgg ggt gaa gat cta aga cat tta ata gta tca aga agt aca     912
cag gca cca cta ata atc aga cct gat tct gga aac cct ctt gac act     960
gtg tta aag gtt ttg gag att tta ggt aag aag ttt cct gtt act gag     1008
aac tca aag ggt tac aag ttg ctg cca cct tat ctt aga gtt att caa     1056
ggg gat gga gta gat att aat acc tta caa gag att gta gaa ggc atg     1104
aaa caa aaa atg tgg agt att gaa aat att gcc ttc ggt tct ggt gga     1152
ggt ttg cta cag aag ttg aca aga gat ctc ttg aat tgt tcc ttc aag     1200
tgt agc tat gtt gta act aat ggc ctt ggg att aac gtc ttc aag gac     1248
cca gtt gct gat ccc aac aaa agg tcc aaa aag ggc cga tta tct tta     1296
cat agg acg cca gca ggg aat ttt gtt aca ctg gag gaa gga aaa gga     1344
gac ctt gag gaa tat ggt cag gat ctt ctc cat act gtc ttc aag aat 1392
ggc aag gtg aca aaa agc tat tca ttt gat gaa ata aga aaa aat gca 1440
cag ctg aat att gaa ctg gaa gca gca cat cat tag 1476

<210> 2
<211> 491
<212> PRT
<213> human

<400> 2

<210>  3
<211>  19
<212>  DNA
<213>  artificial

<220>
<223>  shRNA for NAMPT

<400>  3
gcttcactct gaagagttt                                                    19

## Description

The present invention relates to the use of a vitamin B3 and compounds derived therefrom as well as to nicotinamide phosphorribosyltransferase (NAMPT) and derivatives thereof as well as enhancers or inducers of NAMPT expression, NAMPT activity or NAMPT-mediated signalling for preventing or treating all types of cytopenia of the myeloid or lymphoid lineage. In a particular embodiment, the present invention relates to the use of the above referenced compounds for treating neutropenia, like severe congenital neutropenia, other forms of congenital and acquired neutropenia or chemotherapy-induced febrile neutropenia. In another aspect, the present invention relates to pharmaceutical compositions containing as active ingredients vitamin B3 and metabolites thereof or NAMPT and modulators of the NAMPT activity, NAMPT expression or NAMPT mediated signalling in a combination with a therapeutically active and therapeutically suboptimal amount of G-CSF.

### Prior art

Bone marrow failure syndromes are characterized by a deficiency of one or more hematopoietic lineage. A common feature of both congenital and acquired forms of bone marrow failure syndromes is a marked propensity to develop various types of leukemia, for example, acute myeloid leukemia (AML), acute lymphoblastic leukemia (ALL) or a myelodisplastic syndrome (MDS). The myelodysplastic syndrome (MDS) also as known as preleukemia, represents a diverse collection of hematological conditions united by ineffective production of blood cells and varying risks of transformation to acute myeloid leukemia. The anomalies include neutropenia and thrombocytopenia, abnormal granules in cells, abnormal nucleoshape and size etc. It is sought that MDS arises from mutations and a multipotent bone marrow of stem cells, but the specific defects responsible for these diseases is remained poorly understood. As indicated above, various types of cytopenia including leukopenia may occur in MDS patients. The two serious complications in MDS patients resulting from their cytopenias are bleeding or infection.

Further, various diseases are connected with or result from a "differentiation block" at various immature stages of hematopoietic differentiation, e.g, stages of myelopoiesis. Typical diseases associated with "differentiation block" are neutropenias characterized in a blockage of the differentiation into mature granulocytes, in particular, neutrophilic granulocytes. For example, severe congenital neutropenia (SCN) is a congenital bone marrow failure syndrome characterized by severe neutropenia present from birth, an arrest of myeloid differentiation at the promyelocyte/myelocyte stage, a lack of peripheral blood neutrophils and frequent severe bacterial infections. SCN is considered to be a pre-leukemic syndrome, because more than 20 percent of patients with SCN progress to acute myelogenous leukemia (AML) or MDS. All individuals with CN have a characteristic bone marrow phenotype that distinguishes this condition from other neutropenias: "maturation arrest" with accumulation of granulocyte precursors (promyelocytes) and absence of mature granulocytes. The arrested SCN promyelocytes show impaired proliferation and differentiation in response to granulocyte colony-stimulating factor (G-CSF), as well as accelerated apoptosis. SCN has been described as a heterogeneous disorder involving mutations in various genes such as ELA2, HAX1, G6PC3. G-CSFR, GFI-1, and WASP. Congenital neutropenia follows an autosomal dominant or autosomal recessive pathway of inheritance. In the beginning, bone marrow transplantation from HLA compatible donors was the only curative treatment option for congenital neutropenia patients. Both, the prognosis and the quality of life of congenital neutropenia patients, improved dramatically following the introduction of granulocytes colony-simulating factor (G-CSF) therapy in 1987. More than 90 % of congenital neutropenia patients responded to G-CSF treatment with an increase in the absolute neutrophil counts (ANC).

Hematopoietic stem cell transplantation remains the only currently available treatment for those patients refractory to G-CSF and patients were transformed into AML/MDS. The diagnosis of SCN is usually in infancy for the first month of life because of recurrent severe infections. Beside a decrease of granulocytes, there is usually an increase in blood monocytes from two or four times over normal and increase in the blood eosinophil count is also common.

Neutrophilic granulopoiesis as part of the myelopoiesis, is a life-long multistage process with continues generation of large number of mature neutrophils (> 10⁸ cells per minute per kilogram bodyweight) from a small number of hematopoietic stem cells. To maintain continues constitutive granulopoesis, all these events must be closely regulated by varieties of intrinsic transcription factors, such as RUNX, PU.1, C-EBPalpha and C-EBPbeta and defined cytokines (e.g. G-CSF, GMCSF, IL-3) as well as other metabolites. G-CSF is widely used for the lifelong treatment of patients with congenital neutropenia or chemotherapy-induced febrile neutropenia and for hematopoietic stem cell mobilization.

G-CSF treatment increases blood neutrophil numbers in more than 90% of individuals with congenital neutropenia. A definitive mechanism for this G-CSF-induced differentiation remains unknown. The levels of G-CSF messenger RNA in mononuclear cells, the concentrations of biologically active G-CSF in serum and the expression of G-CSF receptor (G-CSFR) in myeloid cells are considerably elevated in individuals with congenital neutropenia compared with healthy individuals. However, daily injections of pharmacological doses of G-CSF (100-1,000 times higher than physiological levels) are needed to increase neutrophil counts in individuals with congenital neutropenia.

Recently, it was reported that lymphoid enhancer factor-1 (LEF-1) is a crucial transcription factor in the induction of granulopoiesis. LEF-1 activates CCAAT/enhancer binding protein-α (C/EBP-α), a granulocyte-specific transcription factor responsible for steady-state granulopoiesis. LEF-1 and C/EBP are absent in granulocytic progenitors from individuals with congenital neutropenia, suggesting that another granulocyte-specific transcription factor/s promotes G-CSF-triggered granulopoiesis in these individuals.

NAMPT is an enzyme that converts nicotinamide to nicotinamide mononucleotide, which is converted into nicotinamide adenine dinucleotide (NAD⁺) by nicotinamide mononucleotide adenylyltransferase in the mammalian biosynthetic pathway. NAMPT is the rate-limiting factor in NAD⁺ biosynthesis. NAD⁺ and its phosphorylated and reduced forms are hydride-accepting and hydride-donating coenzymes in redox reactions, with central roles in cellular metabolism and energy production. NAD⁺ also activates sirtuins (SIRTs), which are lysine deacetylases for histones and numerous transcription factors. NAMPT localizes to and functions in both intra- and extracellular compartments. Intracellular NAMPT generates NAD and, consequently, has cell-protective benefits. NAMPT was originally identified as a gene product expressed in activated peripheral human lymphocytes and was implicated in the maturation of B cell precursors, and hence was named pre-B cell enhancing factor. Subsequent studies revealed that NAMPT is upregulated during neutrophil activation and acts as an antiapoptotic factor for neutrophils in clinical and experimental sepsis. Increased NAMPT levels are observed in stimulated monocytes and during all-*trans*-retinoid acid (ATRA) induced granulocytic differentiation of the myeloid leukemia cell line HL-60. The cytokines interleukin-1β (IL-1β), tumor necrosis factor and IL-6 induce NAMPT expression. NAMPT is a rate-limiting enzyme for the generation of NAD⁺, which is crucial for SIRT1 activation and regulation of transcription.

Vitamin B3, also known as nicotinamide, is a water-soluble vitamin, is a derivative of pyridine, with a carboxamide group (CONH₂) at the 3-position. Nicotinamide is converted to NAD and NADP in vivo. Vitamin B3 is a precursor to NADH, NAD, NAD+, NADP and NADPH, which play essential metabolic roles in living cells.

In the art, there is still an ongoing demand on providing suitable means for preventing or treating of cytopenia, in particular, cytopenia of myeloid lineage, for example for a treatment of neutropenia like chemotherapy-induced febrile neutropenia, severe congenital neutropenia, or other forms of congenital and acquired neutropenia. In addition, there is still a need for alternatives of a G-CSF monotherapy, alternatives for G-CSF induced stem cell mobilisation and alternatives in the treatment of cancer diseases or disorders, for example of all types of leukaemia.

The present inventors found that G-CSF triggered differentiation of granulocytes is dependent on NAMPT and its product NAD⁺. It is also demonstrated herein that treatment of e.g. healthy individuals with vitamin B3 (nicotinamide), a substrate of NAMPT or *in vitro* treatment of CD34⁺ hematopoietic progenitor cells with vitamin B3 (nicotinamide), induced granulocyte differentiation. Moreover, treatment of patients with severe congenital neutropenia with combination of Vitamin B3 and G-CSF in a dose which is two-fold lower as a regular therapeutic dose (2,5 µg/kg/day instead of 5.0 µg/kg/day) resulted in a dramatic increase of neutrophilic granulocytes in peripheral blood. Additionally, treatment of healthy individuals with vitamin B3 (nicotinamide) resulted in mobilisation of CD34⁺ hematopoietic progenitor cells into peripheral blood.

### Summary of the Invention

In a first aspect, the present invention provides nicotinic acid, nicotinamide, nicotinamide adenine mononucleotide, nicotinamide adenine dinucleotide or derivatives thereof as active Ingredients for preventing or treating of all types of cytopenia of the myeloid or lymphoid lineage or induction/stimulation of granulocytic differentiation.

In another aspect, the present invention provides nicotinamide phosphoribosyltransferase (NAMPT) or a functional fragment or a homolog thereof, or an enhancer or inducer of NAMPT expression, NAMPT activity or NAMPT-mediated signaling as an active ingredient for preventing or treating all types of cytopenia of the myeloid or lymphoid lineage induction/stimulation of granulocytic differentiation.

Preferably, said cytopenia is a cytopenia of the myeloid lineage, in particular, neutropenia, especially severe congenital neutropenia or chemotherapy-induced febrile neutropenia or other congenital or acquired neutropenia.

In a further aspect, the present invention relates to inhibitors of NAMPT expression and/or NAMPT mediated signalling for preventing or treating leukaemia, in particular for treating or preventing acute myeloid leukaemia (AML), for example of cancer of a myeloid lineage with transformed myeloid progenitor cells.

The present invention further provides methods for treatment of individuals suffering from cytopenia, e.g. severe congenital neutropenia or chemotherapy-induced febrile neutropenia or other congenital or acquired neutropenia, thus, the compounds according to the present invention support chemotherapy In an individual.

Another preferred embodiment of the present Invention relates to the provision of a pharmaceutical composition comprising as active ingredients therapeutically active amounts of nicotinic acid, nicotinamid, nicotinamid adenine mononucleotide, nicotinamide adenine dinucleotide or derivatives thereof in combination with G-CSF and, optionally, a pharmaceutically acceptable carrier.

Furthermore, the present invention provides a pharmaceutical composition comprising as active ingredients therapeutically effective amounts of NAMPT or a functional fragment or homolog thereof, or an enhancer or inducer of NAMPT expression, NAMPT activity or NAMPT-mediated signalling alone or in combination with G-CSF, and, a pharmaceutically acceptable carrier.

In addition, the present invention relates to methods for mobilizing hematopoietic stem cells, in particular, human hematopoietic stem cells and/or Inducing differentiation or expansion (proliferation) of said stem cells or pluripotent hematopoietic progenitor cells into the myeloid lineage comprising contacting said cells with nicotinic acid, nicotinamid, nicotinamide adenine monocucleotide, nicotinamide adenine dinucleotide or derivatives thereof or with NAMPT or a functional fragment or homolog thereof, or an enhancer or inducer of NAMPT expression, NAMPT activity or NAMPT-mediated signalling alone or in combination with G-CSF.

### Brief description of the drawings

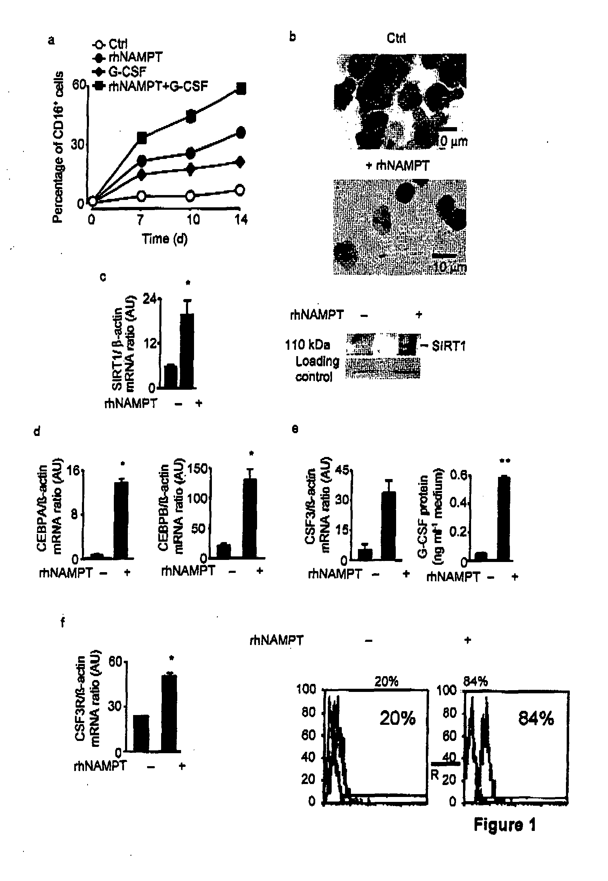

**Figure 1****.** NAMPT triggers myeloid differentiation of CD34⁺ cells from healthy individuals. CD34⁺ cells were cultured from healthy individuals (n = 3) with 10 ng ml⁻¹ G-CSF, 10 ng ml⁻¹ NAMPT or a combination of both. (**a**) Granulocytic (CD16) surface marker expression, as measured by FACS; data represent means ± s.d. and are derived from three independent experiments each In triplicate. (**b**) Wright-Giemsa-stained images of cytospin preparations from treated CD34⁺ cells on day 14 of culture. (**c**) mRNA and protein expression of SIRT1 in CD34⁺ cells incubated with or without NAMPT. Data represent means ± s.d. and are derived from three independent experiments, each in triplicate. **P* < 0.05. (**d**) C/EBP-α and C/EBP-β mRNA expression of CD34⁺ cells incubated with or without NAMPT measured on the fourth day of culture; data represent means ± s.d. and are derived from three independent experiments, each in triplicate. **P* < 0.05. (**e**) G-CSF mRNA expression and protein secretion in culture supernatants of CD34⁺ cells incubated with or without NAMPT, measured by G-CSF-specific ELISA. Data represent means ± s.d. and are derived from three independent experiments, each in triplicate. **P* < 0.05; ***P* < 0.01. (f) G-CSFR mRNA expression and representative histogram of G-CSFR surface expression measured by FACS on day 4 of culture (black line represents isotype control and red line represents G-CSFR staining). Data represent means ± s.d. and are derived from three independent experiments, each in triplicate. **P* < 0.05.

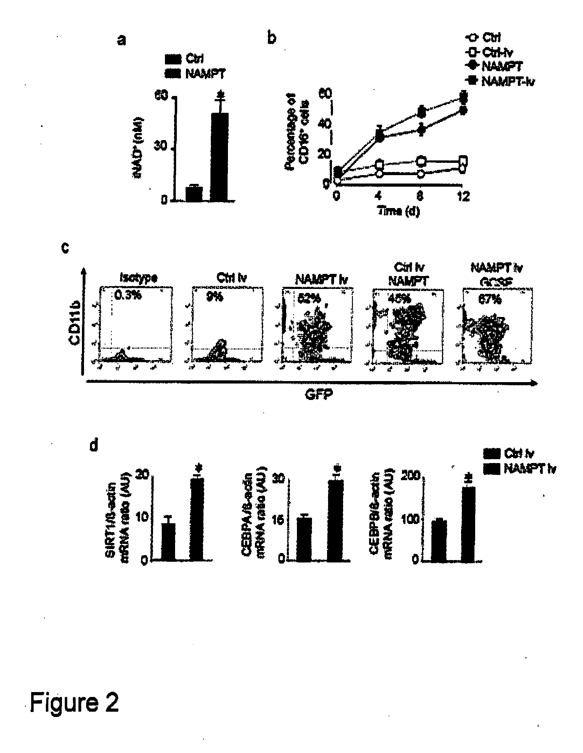

**Figure 2****. NAMPT Induces myeloid differentiation of CD34⁺ cells from healthy individuals**
   CD34⁺ cells were cultured from healthy individuals (*n* = 3) with 10 ng/ml of G-CSF, or 10 ng/ml NAMPT or in combination of both. Alternatively, we transduced CD34⁺ cells from healthy individuals (n = 3) with lentiviral constructs contained NAMPT-GFP (NAMPT Iv), or only GFP (ctrl Iv), positively sorted GFP⁺ cells and cultured them, as described in Material and Methods. (**a**) intracellular levels of NAD⁺ (iNAD⁺) from CD34⁺ cells incubated without or with NAMPT measured on 4 day of culture, data represent means ± s.d. and are derived from three independent experiments each in triplicate, *, *P* < 0.05; (**b**) granulocytic (CD16) surface marker expression by FACS and presented as percentage of CD16⁺ cells, data represent means ± s.d. and are derived from three independent experiments each in triplicate; (**c**) representative data from FACS analysis of NAMPT-GFP Iv or ctrl Iv transduced CD34⁺ cells incubated with or without NAMPT or G-CSF and stained with CD11b-specific PE conjugated antibody; (**d**) mRNA expression of indicated genes by qRT-PCR. Data represent means ± s.d. and derived from three independent experiments each in triplicate (*, *P* < 0.05; **, *P* < 0.01).

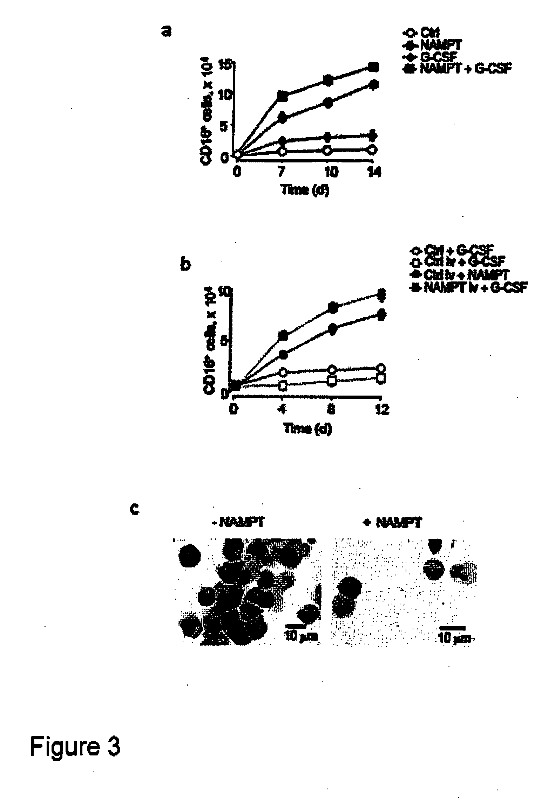

**Figure 3****. NAMPT induces myeloid differentiation of CD34⁺ cells from CN patients**
   (**a**) CD34⁺ cells were incubated from CN patients (n = 2) without or with NAMPT, or G-CSF, or with combination of both, we measured CD16 surface expression at different time points using FACS, as described in Materials and Methods, percentage of CD16⁺ cells is depicted, data represent means ± s.d. and are derived from two independent experiments each in triplicate; (**b**) CD34⁺ cells were transduced from CN patients (n = 2) with lentiviral constructs contained NAMPT-GFP (NAMPT Iv), or only GFP (ctrl Iv), positively sorted GFP⁺ cells and cultured with G-CSF, or with NAMPT, we measured CD16 surface expression at different time points using FACS, percentage of CD16⁺ cells is depicted, data represent means ± s.d. and are derived from two independent experiments each in triplicate; (**c**) Wright-Giemsa stained images of cytospin preparations of CD34⁺ cells from CN patients incubated without or with NAMPT, assessed on day 14 of culture.

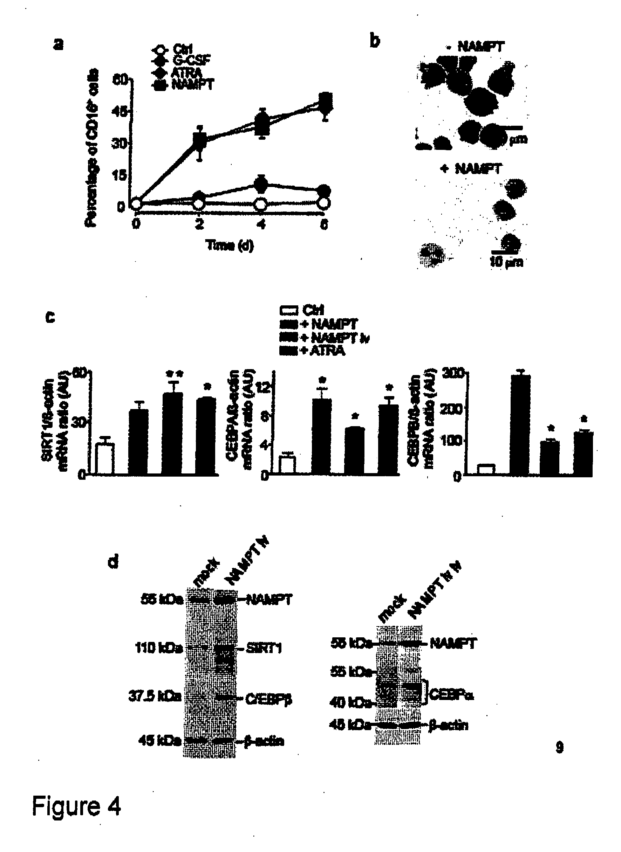

**Figure 4****. NAMPT triggeres myeloid differentiation of the myeloid leukemia cell line HL-60**
   HL-60 cells were cultured with ATRA, G-CSF, or NAMPT. Alternatively, HL-60 cells were transduced with lentiviral constructs contained NAMPT-GFP (NAMPT Iv), or only GFP (ctrl Iv). (**a**) granulocytic (CD16) surface marker expression was assessed by FACS, percentage of CD16⁺ cells is presented, data represent means ± s.d, and are derived from three independent experiments each in triplicate; (**b**) Wright-Giemsa stained images of cytospin preparations from treated cells on day 10 of culture; (**c**) SIRT1, CEBPA and CEBPB mRNA expression of treated or transduced cells on day 4 of culture, data represent means ± s.d. and are derived from three independent experiments each in triplicate, *, *P* < 0,05; (**d**) protein expression of indicated proteins assessed by Western blot.

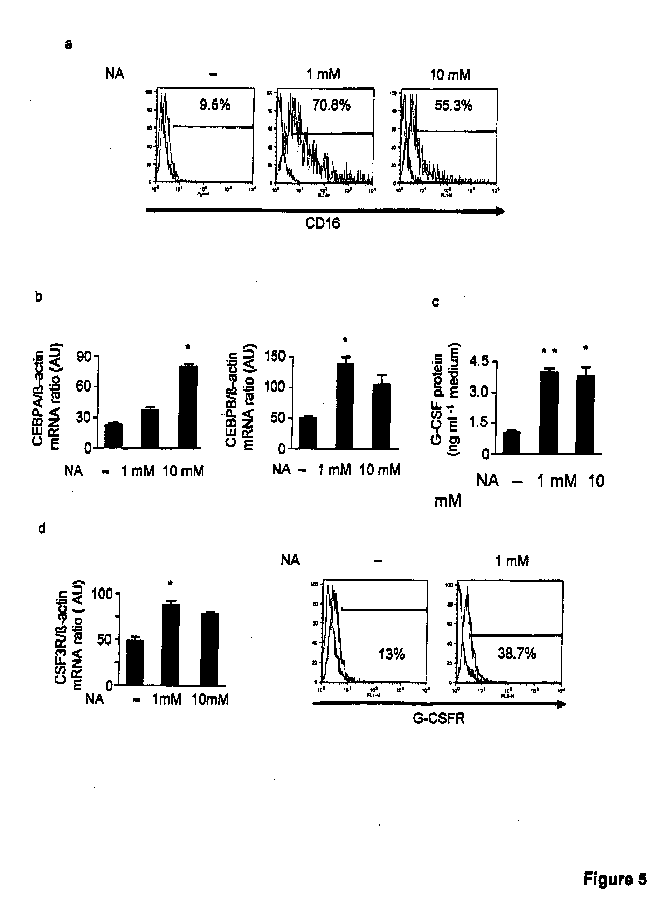

**Figure 5****.** Nicotinamide induces *in vitro* myeloid differentiation of CD34⁺ cells from healthy individuals.
   CD34⁺ cells were cultured from healthy individuals (n = 3) with 1 mM or 10 mM nicotinamide. (**a**) CD16 granulocytic surface marker expression, as measured on day 7 of culture by FACS and depicted as representative histogram images. (**b**) C/EBP-α and C/EBP-β mRNA expression of CD34⁺ cells incubated with or without with NAMPT measured on the fourth day of culture. Data represent means ± s.d. and are derived from three independent experiments, each in triplicate. **P* < 0.05. (**c**) G-CSF protein abundance in culture supernatants of CD34⁺ cells incubated with or without nicotinamide, as measured by G-CSF-specific ELISA. Data represent means ± s.d. and are derived from three independent experiments, each in duplicate. **P* < 0.05; ***P* < 0.01. (**d**) G-CSFR mRNA expression and a representative histogram of G-CSFR surface expression, as measured by FACS on the fourth day of culture (black line represents isotype control and red line represents G-CSFR staining). Data represent means ± s.d, and are derived from three independent experiments, each in triplicate. **P* < 0.05.

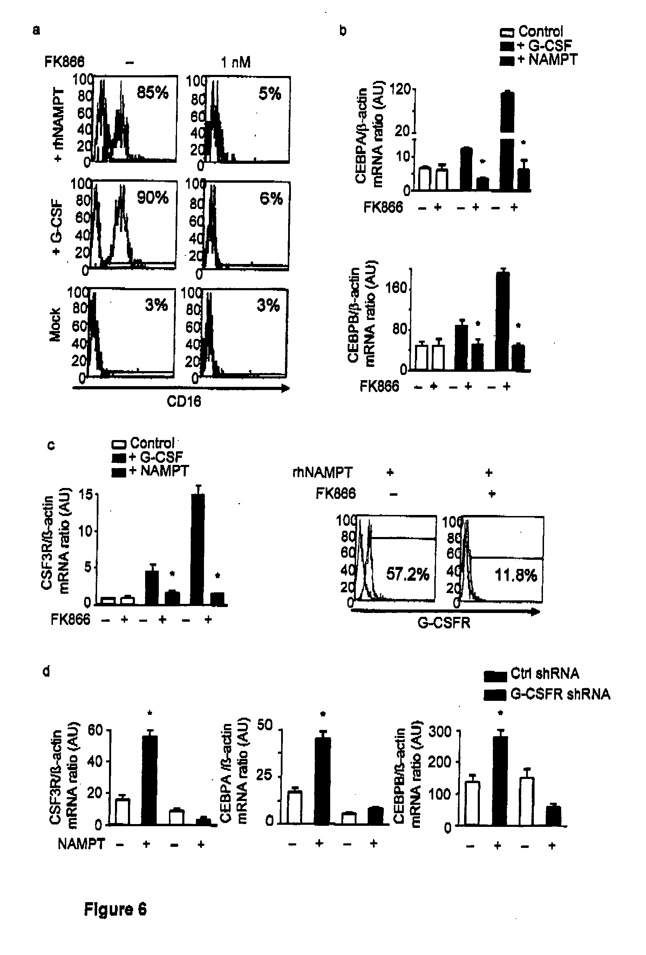

**Figure 6****.** NAMPT is capable of G-CSF-dependent granulocytic differentiation. (a,b) CD34⁺ hematopoietic progenitor cells were treated with 1 nM FK866, subsequently stimulated the cells with either G-CSF or NAMPT and evaluated granulocytic (CD16) surface marker expression by FACS (**a**) and mRNA expression of C/EBP-α and C/EBP-β by quantitative RT-PCR (**b**). Data represent means ± s.d, and are derived from three independent experiments, each in triplicate. **P* < 0.05. (**c**) G-CSFR mRNA and protein expression, as determined by quantitative RT-PCR and FACS (representative histogram is depicted; black line represents isotype control and red line represents G-CSFR staining), respectively. Data represent means ± s.d. and are derived from three independent experiments, each in triplicate. **P* < 0.05. (**d**) We transduced CD34⁺ cells with control red-fluorescent protein (RFP) or G-CSFR-RFP shRNA and measured G-CSFR, C/EBP- α and C/EBP-β mRNA expression of sorted cells on day 4 of culture. Data represent means ± s.d. and are derived from three independent experiments, each in triplicate. **P* < 0.05.

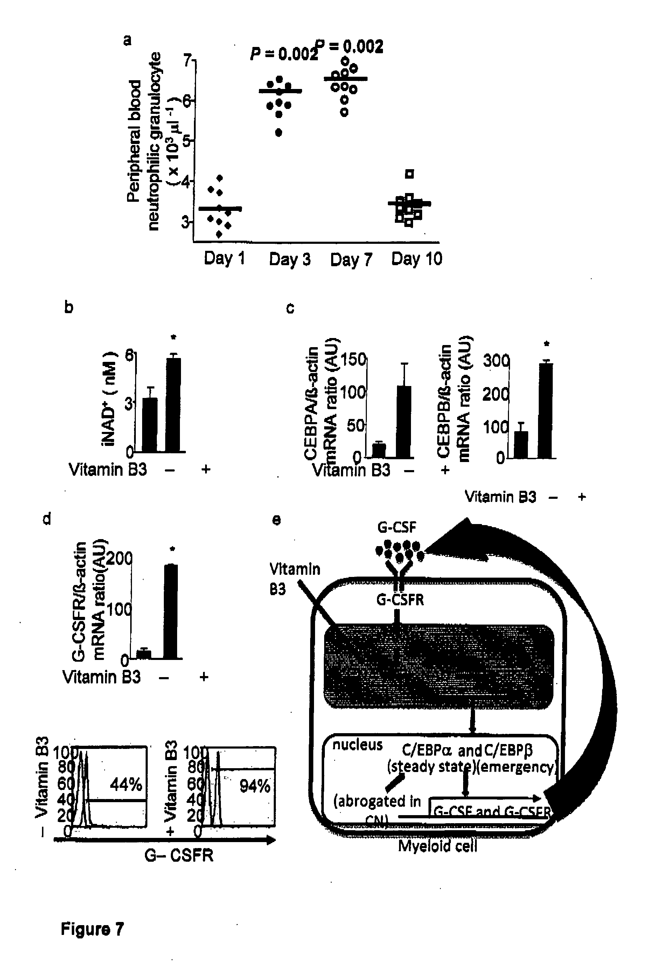

**Figure 7****.** Treatment of healthy individuals with vitamin B3 leads to increased numbers of neutrophils in peripheral blood.
   Six healthy individuals were treated with vitamin 83 (10 - 20 mg per kg per d) for 1 week and analyzed blood cells daily. To document the reproducibility of the responses, three individuals were treated twice in two independent experiments. (**a**) The number of neutrophilic granulocytes before (day 0), during (day 3 and day 7) and after (day 10) vitamin B3 treatment. (**b**) Concentration of iNAD⁺ in peripheral blood PMNs from vitamin B3-treated healthy individuals, as compared to the untreated state. Data represent means ± s.d. of triplicates. **P* < 0.05. (**c**) C/EBP-α and C/EBP-β mRNA expression in CD33⁺ myeloid cells, as determined by quantitative RT-PCR on day 3 of vitamin B3 treatment. Data represent means ± s.d. of triplicates. **P* < 0.05. (**d**) G-CSFR mRNA expression and a representative histogram of G-CSFR surface expression in peripheral blood PMNs, as measured by FACS on day 3 of vitamin B3 treatment (black line represents isotype control and red line represents G-CSFR staining). Data represent means ± s.d. of triplicates. **P* < 0.05. (**e**) Mechanism of NAMPT and vitamin B3 effects on granulopoiesis. G-CSFR activation upon ligand binding induces NAMPT mRNA and protein synthesis, with subsequent elevation of NAD⁺ concentrations and NAD⁺-dependent SIRT1 activity. Increased SIRT1 activity in turn leads to elevated C/EBP-α and/or C/EBP-β dependent G-CSFR and G-CSF synthesis, with a subsequent induction of granulopoiesis via an autoregulatory feedback loop. C/EBP-α regulates steady-state granulopoiesis, which is abrogated in individuals with congenital neutropenia, and C/EBPβ is responsible for emergency granulopoiesis. CN, congenital neutropenia.
**Figure 8****.**
   One patient suffering from severe congenital neutropenia was treated with 2,5 µg/kg/day of G-CSF alone or in combination of Vitamin B3 (20 mg/kg/day) and G-CSF (2,5 µg/kg/day). The number of leukocytes and absolute number of neutrophilic granulocytes was measured at indicated time points.

### Detailed description of the present invention

The present inventors recognized that NAMPT is crucial in granulocytopoiesis and, e.g. the activity of NAMPT and the presence of the product of NAMPT, namely, NAD⁺, is elevated in cytopenia, in particular cytopenia of the myeloid or lymphoid lineage, like neutropenia, in particular, severe congenital neutropenia or chemotherapy-induced febrile neutropenia or other congenital or acquired neutropenia.

In a first embodiment, the present invention relates to the use of nicotinic acid, nicotinamide, nicotinamide adenine monocucleotide, nicotinamide adenine dinucleotide or derivatives thereof for preventing or treating all types of cytopenia of the myeloid or lymphoid lineage.

In the present application, the term "derivatives" refers to compounds of nicotinic acid, nicotinamide, nicotinamide adenine monocucleotide, nicotinamide adenine dinucleotide having chemical modifications without interfering with the properties of being a substrate or product of NAMPT.

In another aspect, the present invention relates to the use of NAMPT, a functional fragment or homolog thereof, or an enhancer or inducer of NAMPT expression, NAMPT activity or NAMPT-mediated signalling for preventing or treating cytopenia in an individual suffering therefrom.

In a preferred embodiment, said cytopenia is a cytopenia of the lymphoid or myeloid lineage. In particular, said cytopenia is a cytopenia of the myeloid lineage, in particular, neutropenia.

In a particular preferred embodiment, the neutropenia is a severe congenital neutropenia or chemotherapy-induced febrile neutropenia or other congenital or acquired neutropenia.

Thus, in a further aspect, the present invention relates to the use of nicotinic acid, nicotinamide, nicotinamide adenine monocucleotide, nicotinamide adenine dinucleotide or derivatives thereof or NAMPT, a functional fragment or homolog thereof, or an enhancer or Inducer of NAMPT expression, NAMPT activity or NAMPT-mediated signalling for preventing or treating cytopenia in an individual suffering therefrom for supporting chemotherapy, namely, reducing or suppressing the risk of developing febrile neutropenia or other types of diseases due to suppressed granulocytopoiesis.

The use of the compounds according to the present invention allows to fasten the regeneration of the granulocytes following chemotherapy of cancer patients. Furthermore, the compounds of the present invention have the ability to mobilize hematopoietic stem cells into peripheral blood and to promote the differentiation of said stem cells into granulocytes and other types of cells of the myeloid lineage.

In another embodiment, the present invention relates to a method for preventing or treating cytopenia of the myeloid or lymphoid lineage in an individual comprising the step of administering either nicotinic acid, nicotinamide, nicotinamide adenine monocucleotide, nicotinamide adenine dinucleotide or derivatives thereof, or NAMPT or a functional fragment or homolog thereof, or an enhancer or an inducer of NAMPT expression, NAMPT-mediated signalling for preventing or treating cytopenia In an individual suffering NAMPT activity or NAMPT-mediated signalling in a therapeutically effective amount to an individual in need thereof. In particular, said method relates to preventing or treating cytopenia of the myeloid lineage, like neutropenia, in particular, severe congenital neutropenia or chemotherapy-induced febrile neutropenia or other congenital or acquired neutropenia.

In a further aspect, the present invention relates to a method for promoting proliferation of cells of the myeloid lineage, e.g. precursors of neutrophils or granulocytes, like promyelocytes, comprising the step of administering nicotinic acid, nicotinamide, nicotinamide adenine monocucleotide, nicotinamide adenine dinucleotide or derivatives thereof or NAMPT or a functional fragment or homolog thereof, or an enhancer or an inducer of NAMPT expression, NAMPT activity or NAMPT-mediated signalling in a therapeutically effective amount to an individual in need thereof to the cells *in vivo* or *in vitro*.

The NAMPT molecule maybe in form of a protein, e.g. according to Seq. ID No.2 (GeneBank Accession No. AAI06047) or a peptide or in form of a nucleic acid molecule, e.g. Seq. ID No, 1 according to GeneBank Accession No. NM_005746.

In a preferred embodiment, the inducer or enhancer of NAMPT expression, NAMPT activity or NAMPT-mediated signalling is a molecule enhancing transcription of a nucleic acid molecule encoding NAMPT or a molecule extending the half-life of the protein or stabilizing the mRNA in a cell.

The term "a functional fragment thereof" as used herein, refers to a fragment of the NAMPT protein, e.g. a peptide, or the nucleic acid encoding NAMPT, e.g. the nucleic acid according to Seq. ID No. 1 derived from GeneBank Accession No. NM_005746 displaying the same activity, namely, converting nicotinamide to nicotinamide mononucleotide.

In the present document, the term NAMPT includes NAMPT fragments and homologs unless otherwise indicated. For example, the NAMPT protein is the protein according to Seq. ID No. 2 corresponding to GeneBank Accession No. AAI06047.

The term "homolog" as used herein, refers to molecules having NAMPT activity. In particular, a homolog has the same activity as NAMPT in the NAMPT mediated signalling pathway, namely converting nicotinamide to nicotinamide mononucleotide.

As used herein, the term "individual" or "subject" is used herein interchangeably and refers to an individual or a subject in need of a therapy or prophylaxis or suspective to be afflicted with a condition or disease mentioned herein. Preferably, the subject or individual is a mammal, in particular preferred, a human.

The activity of NAMPT may be determined by methods known in the art, e.g. by PCR, western blot, northern blot, ELISA or other methods known in the art.

In a further aspect, the present invention relates to an inhibitor of NAMPT expression NAMPT and/or NAMPT-mediated signalling alleviating or interrupting the NAMPT activity, namely, converting nicotinamide to nicotinamide mononucleotide, thus, alleviating or interrupting the amount of nicotinamide mononucleotide or/and nicotinamide dinucleotide present in a cell.

In particular, the inhibitor allows treating or preventing leukemia, like ALL, AML or CML. In case of myeloid leukemia, the inhibitor of NAMPT expression or NAMPT signalling enables treating cancer of the myeloid lineage with transformed myeloid precursors, in particular, acute myeloblastic leukemia, acute promyelocytic leukemia, acute myelomonocytic leukemia and acute monoblastic leukemia as well as chronic myeloblastic leukemia.

Thus, the present invention refers in another aspect to a method for treating individuals afflicted with the above mentioned diseases comprising the step of administering an inhibitor of NAMPT expression, NAMPT activity and/or NAMPT mediated signalling. For example, the inhibitor is FK866 obtained from the National Institute of Mental Health, Chemical Synthesis and Drug Supply Program, Dr. Ken Rehder.

According to the present invention, the compounds and pharmaceutical composition described herein may be used as an alternative for G-CSF or may be used in combination with G-CSF.

In a preferred embodiment, nicotinic acid, nicotinamide, nicotinamide adenine mononucleotide, nicotinamide adenine dinucleotide or derivatives thereof are used or administered in an amount of a daily dosage in the range of 1 to 50 mg/kg bodyweight, preferably, 2 to 30 mg, like 5 to 20 mg, in particular, 10 to 20 mg/kg bodyweight.

That is, a preferred embodiment relates to a method for treating or preventing cytopenia of the myeloid or lymphoid lineage in an individual comprising step of administering nicotinic acid, nicotinamide, nicotinamide adenine mononucleotide, nicotinamid adenine dinucleotide or derivatives thereof in an therapeutically effective amount to an individual in need thereof, preferably, for preventing or treating cytopenia of the myeloid lineage, like for preventing or treating neutropenia, in particular severe congenital neutropenia or chemotherapy-induced febrile neutropenia or other congenital or acquired neutropenia.

In another embodiment, the invention concerns a method of treating or preventing defective myelopoiesis in an individual comprising step the of adiminstering nicotinic acid, nicotinamide, nicotinamide adenine mononucleotide, nicotinamid adenine dinucleotide to an individual suffering therefrom.

The method may further comprise the step of administering a therapeutically effective amount of G-CSF.

In another aspect, the present invention relates to a method for differentiating granulocytes in-vivo or in-vitro comprising the step of administering an effective amount of nicotinic acid, nicotinamide, nicotinamide adenine mononucleotide, nicotinamid adenine dinucleotide to precursor cells of said granulocytes, e.g. the method further comprises the step of administering an effective amount of G-CSF.

Preferably, the method according to the present invention comprises administering an amount of G-CSF to the individual in need thereof which is at least two times lower than the effective dosage administered when treating said individual with G-CSF alone.

That is, the present invention allows to reduce the dosage of G-CSF to be administered to the patent to suboptmial doses. According to the present invention, the term "suboptimal doses" refers to doses of G-CSF which are not therapeutically effective when given alone. For example, the suboptimal doses is at least two times lower than the therapeutically effective doses, e.g. the suboptimal doses is 1 µg/kg bodyweight/day while the therapeutically effective doses is 10 µg/kg bodyweight/day.

Moreover, the present invention relates to a method for mobilising and differentiating hematopoietic stem cells. Alone or in combination with low doses of G-CSF (at least two times lower than the effective dosage administered when treating said individual with G-CSF alone), the compounds according to the present inventing allows to mobilise and differentiate the stem cells in the lymphoid and myeloid lineage.

Thus, in the method for preventing or treating cytopenia according to the present invention, optionally, a therapeutically effective amount of G-CSF is administered to said individual. The combination of the compounds according to the present invention with G-CSF allows to increase the numbers of granulocytes and/or to decrease the required therapeutic doses of G-CSF in the individual suffering from cytopenia, in particular, neutropenia. Particularly, this is of economic and therapeutic interest since treatment with G-CSF requires daily injection of G-CSF and the medical costs for G-CSF treatment are very high.

In contrast, nicotinic acid, nicotinamide, nicotinamide adenine monocucleotide, nicotinamide adenine dinucleotide or derivatives thereof are inexpensive. For example, nicotinic acid, or vitamin B3, is produced at low costs and available in amounts of tons.

It has been recognized by the present inventors that NAMPT plays a crucial role *inter alia* In granulocytopoiesis, in particular, of neutrophil granulocytopoiesis. Thus, on the one hand, interrupting the NAMPT signalling pathways allows reducing or inhibiting excessive proliferation, survival and differentiation of granulocyte progenitor cells. On the other hand, fostering and elevating the NAMPT level alone on in combination with corresponding growth factors (e.g. G-CSF to induce granulopoiesis) and providing the educts or products of NAMPT alone or in combination with the corresponding growth factor mentioned before, enables overcoming cytopenia of the myeloid and lymphoid lineage, in particular, overcoming a maturation arrest in the differentiation of progenitor cells of granulocytes.

The pharmaceutical composition according to the present invention comprises the NAMPT molecule or an inducer or enhancer of the NAMPT molecule as described herein or in case of a pharmaceutical composition for treating cancer, the inhibitor of NAMPT as described herein and, optionally, a pharmaceutical acceptable carrier. Such pharmaceutical compositions comprise a therapeutically effective amount of the conjugates and, optionally, a pharmaceutically acceptable carrier. The pharmaceutical composition may be administered with a physiologically acceptable carrier to a patient, as described herein. Acceptable means that the carrier be acceptable in the sense of being compatible with the other ingredients of the composition and not be deleterious to the recipient thereof. In a specific embodiment, the term "pharmaceutically acceptable" means approved by a regulatory agency or other generally recognized pharmacopoeia for use in animals, and more particularly in humans. The term "carrier" refers to a diluent, adjuvant, excipient, or vehicle with which the therapeutic is administered. Such pharmaceutical carriers can be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously, Saline solutions and aqueous dextrose and glycerol solutions can also be employed as liquid carriers, particularly for injectable solutions. Suitable pharmaceutical excipients include starch, glucose, lactose, sucrose, gelatine, malt, rice, flour, chalk, silica gel, sodium stearate, glycerol monostearate, talc, sodium chloride, dried skim milk, glycerol, propylene, glycol, water, ethanol and the like. The composition, if desired, can also contain minor amounts of wetting or emulsifying agents, or pH buffering agents. These compositions can take the form of solutions, suspensions, emulsion, tablets, pills, capsules, powders, sustained-release formulations and the like. The composition can be formulated as a suppository, with traditional binders and carriers such as triglycerides. Oral formulation can include standard carriers such as pharmaceutical grades of mannitol, lactose, starch, magnesium stearate, sodium saccharine, cellulose, magnesium, carbonate, etc. Examples of suitable pharmaceutical carriers are described in "Remington's Pharmaceutical Sciences" by E.W. Martin (18^{th} ed., Mack Publishing Co., Easton, PA (1990)). Such compositions will contain a therapeutically effective amount of the aforementioned compounds, salts or solvates thereof, preferably in purified form, together with a suitable amount of carrier so as to provide the form for proper administration to the patient. The formulation should suit the mode of administration.

Typically, pharmaceutically or therapeutically acceptable carrier is a carrier medium which does not interfere with the effectiveness of the biological activity of the active ingredients and which is not toxic to the host or patient.

In another preferred embodiment, the composition is formulated in accordance with routine procedures as a pharmaceutical composition adapted for intravenous administration to human beings. Typically, compositions for intravenous administration are solutions in sterile isotonic aqueous buffer. Where necessary, the composition may also include a solubilizing agent and a local anesthetic such as lidocaine to ease pain at the site of the injection. Generally, the ingredients are supplied either separately or mixed together in a unit dosage form, for example, as a dry lyophilised powder or water free concentrate in a hermetically sealed container such as an ampoule or sachette indicating the quantity of active agent. Where the composition is to be administered by infusion, it can be dispensed with an infusion bottle containing sterile pharmaceutical grade water or saline. Where the composition is administered by injection, an ampoule of sterile water for injection or saline can be provided so that the ingredients may be mixed prior to administration.

The pharmaceutical composition for use in connection with the invention can be formulated as neutral or salt forms. Pharmaceutically acceptable salts include those formed with anions such as those derived from hydrochloric, phosphoric, acetic, oxalic, tartaric acids, etc., and those formed with cations such as those derived from sodium, potassium, ammonium, calcium, ferric hydroxides, isopropylamine, triethylamine, 2-ethylamino ethanol, histidine, procaine, etc.

The term "therapeutically or pharmaceutically effective amount" as applied to the compositions of the present invention refers to the amount of compositions sufficient to induce the desired biological result. That result can be alleviation of the signs, symptoms or causes of a disease or any other desired alteration of a biological system. In the present invention, the result will typically involve e.g. decrease or increase of NAMPT expression and, thus, altering the absolute neutrophil count in said individual.

In vitro assays may optionally be employed to help identifying optimal dosage ranges. The precise dose to be employed in the formulation will also depend on the route of administration, and the seriousness of the disease or disorder, and should be decided according to the judgment of the practitioner and each patient's circumstances. Effective doses may be extrapolated from dose-response curves derived from in vitro or animal model test systems. Preferably, the pharmaceutical composition is administered directly or in combination with an adjuvant.

The term "administered" as used herein means administration of a therapeutically effective dosage of the aforementioned pharmaceutical composition to an individual. By "therapeutically effective amount" is meant a dose that produces the effects for which it is administered. The exact dose will dependent on the purpose of the treatment and will be ascertainable by once skilled in the art using known techniques. It is known in the art adjustments for systemic vs. localized delivery, age, body weight, general health, sex, diet, time of administration, drug interaction and severity of the condition may be necessary, and will be ascertainable with routine experimentation by those skilled in the art.

The administration of the pharmaceutical composition can be done in a variety ways including, but not limited to orally, subcutaneously, intravenously, intraarterial, intranodal, intramedulary, intrathecal, intraventricular, intranasaly, intrabronchial, transdermally, intrarectally, intraperitonally, intramuscularly, intrapulmonary, vaginaly or intraoculary.

The present invention allows to treat leukaemia and other malignancies associated with elevated NAMPT levels by inhibiting NAMPT mRNA/protein synthesis, or blocking of NAMPT protein using e.g. NAMPT -specific small inhibitory molecules, inhibitory synthetic peptides or inhibitory RNAs or DNAs or similar nucleic acid like molecules like DNA etc.

In addition, according to the present invention, methods are provided for the treatment of congenital non-malignant disorders of hematopoiesis, e.g. congenital neutropenia, immunodeficiencies due to altered expression and activity of NAMPT. Said methods involve the use of NAMPT -specific stimulatory small molecules of stimulatory synthetic peptides.

Another aspect of the present invention relates to the use of NAMPT, or a fragment or homolog thereof, or an inducer or enhancer of NAMPT expression, activity or NAMPT mediated signalling in the mobilisation and differentiation of stem cells in particular of human stem cells in a fashion similar to the way it is described and well-known in the literature for G-CSF.

These and other embodiments are disclosed and encompassed by the description and examples of the present invention. Further literature concerning any one of the methods, uses and compounds to be employed in accordance with the present invention may be retrieved from public libraries, using for example electronic devices. For example the public database "Medline" may be utilized which is available on the Internet, for example under http://www,ncbi.ncbi.nlm.nih,gov/PubMed/medline.html. Further databases and addresses, such as http://www.ncbi.nim.nih.gov/, http://www.tigr.org/, are known to the person skilled in the art and can also be obtained using, e.g., http://www.google.de. An overview of patent information in biotechnology and a survey of relevant sources of patent information useful for retrospective searching and for current awareness is given in Berks, TIBTECH 12 (1994), 352-384.

Not to be bound by theory, the present invention will be described further by the way of examples. It is clear that said examples are intended to illustrate the present invention further without limiting the invention thereto.

For example, it is shown that G-CSF-triggered differentiation of myeloid precursors is dependent on NAMPT and its product NAD⁺. Notably, treatment of healthy individuals with vitamin B3, a substrate of NAMPT, leads to neutrophilia. These findings support a crucial role of NAMPT and NAD⁺ in myeloid differentiation.

### Examples

The following methods have been applied in the present invention:

### METHODS

Participants In this in vitro study included 13 subjects with severe congenital neutropenia, three subjects with neutropenia associated with congenital disorders of metabolism (one subject with glycogen storage disease type 1 b and two subjects with Shwachman-Diamond syndrome) and two with other types of chronic neutropenia. All of these subjects had received long-term (>1 year) G-CSF treatment; the G-CSF dose ranged between 1.2 and 7.5 µg per kg body weight per d, or once in 2 d. Two of the subjects with congenital neutropenia were examined twice: first before initiation of G-CSF therapy and then during long-term G-CSF treatment. Three healthy volunteers received G-CSF at a dose of 5 µg per kg body weight per d for 3 d. Bone marrow samples were collected in association with the annual follow-up recommended by the Severe Chronic Neutropenia International Registry. Informed consent was obtained from all subjects. Approval for the study was obtained from the Hannover Medical School Institutional Review Board.

Bone marrow and peripheral blood mononuclear cells were isolated by Ficoll-Hypaque gradient centrifugation (Amersham Biosciences) and positively selected bone marrow CD34⁺ and CD33⁺ cells by sequential immunomagnetic labeling with corresponding magnetic cell-sorting beads (Miltenyi Biotech). Peripheral blood PMNs were isolated by Ficoll-Hypaque gradient centrifugation and by subsequent hypotonic lysis of erythrocytes in cell pellet, Cells were counted and viability was assessed by trypan blue dye exclusion. The purity of sorted CD34⁺ cells, CD33⁺ cells and PMNs was more than 96% as assessed by FACS analysis and by staining with May-Grünwald-Giemsa.

Purified PMNs and CD33⁺ cells were cultured in RPMI-1640 medium supplemented with 1% heat-inactivated autologous human serum with or without addition of 10 ng ml⁻¹ of recombinant human G-CSF (rhG-CSF) (Cell Systems Biotechnology). PMNs were cultured in polypropylene tubes (1 x 10⁶ cells per ml per tube) and CD33⁺ cells in 24-well tissue culture plates (Costar; 2 x 10⁵ cells per well). 1 x 10⁶ CD34⁺ cells have been cultured in X-VIVO 10 medium (Cambrex) supplemented with 20 ng ml⁻¹ IL-3, 20 ng ml⁻¹ of IL-6, 20 ng ml⁻¹ of thrombopoietin, 50 ng ml⁻¹ stem cell factor, 50 ng ml⁻¹ Flt3 ligand (Cell Systems Biotechnology) and 0.5% human serum albumin. HL-60 cells (obtained from the German Collection of Microorganisms and Cell Cultures (OSMZ) (1 x 10⁵ cells per ml) have been cultured in RPMI 1640 medium supplemented with 1% FCS. To assess proliferation, viable cells have been counted by trypan blue dye exclusion in a hemocytometer and measured BrdU uptake with the BrdU Flow Kit (Pharmingen). For granulocytic differentiation, we cultured 1 x 10⁵ CD34⁺ were cultured cells in the presence of G-CSF (10 ng ml⁻¹), NAMPT (10 ng ml⁻¹), or nicotinamide (1 mM or 10 mM). For inhibition studies, CD34⁺ cells have been treated with 1 nM NAMPT-specific inhibitor FK866 (National Institute of Mental Health, Chemical Synthesis and Drug Supply Program, USA). Granulocytic differentiation was characterized by FACS analysis of cells stained with antibody specific to CD16 (Immunotools), CD15 (BD Pharmigen) or CD11b (BD Pharmigen) and by morphological assessment of Wright-Giemsa-stained cytospin slides. For assessment of G-CSFR protein expression cells were stained with unconjugated antibody to mouse G-CSFR and secondary FITC-conjugated antibody and measured G-CSFR expression by FACS. We cultured HL-60 cells in the presence of 10 ng ml⁻¹ human rhG-CSF(Cell systems Biotechnoogy), 10 ng ml⁻¹ human rhNAMPT (Phoenix Pharmaceuticals) or 0.1µM ATRA (Sigma).
For the measurement of NAD⁺, 0.5 M ice-cold HClO₄ was added to the cells, or cell culture supernatants or plasma; after 2 min, 100µl of supernatants was collected by centrifugation at 3,000g for 5 min, was 20µl K₂HPO₄ (1 M) added with cooling on ice and adjusted pH to 7.2-7.4 with KOH. 50µl of supernatant was given to the reaction mixture containing 0.1 M sodiumpyrophosphate-semicarbazid (Sigma) (pH 8.8), absolute ethanol and dH₂O, NAD⁺ spectrophotometrically was assessed at 339 nm at room temperature (25°C) as as mean difference in absorbance before (A₁) and 6 min after (A₂) addition of alcohol dehydrogenase (Roche). G-CSF protein was measured by G-CSF-specific ELISA from R&D Systems and NAMPT protein by NAMPT specific carboxy-terminal enzyme immunometric assay (Phoenix Pharmaceuticals).

The following antibodies have been used rabbit polyclonal antibody to PBEF (Phoenix Pharmaceuticals), rabbit polyclonal antibody to SIRT1, rabbit polyclonal antibody to C/EBPα and rabbit polyclonal antibody to C/EBPα (all three from Cell Signaling Technology), mouse monoclonal antibody to β-actin (Santa Cruz Biotechnology) and secondary HRP-conjugated antibody to mouse or rabbit IgG (Santa Cruz Biotechnology), Whole cell lysates were obtained either through lysis of a defined number of cells in lysis buffer (Fermentas) or through direct disruption in Laemmli's loading buffer, followed by brief sonication, Proteins were separated by 10% SDS-PAGE and the blots were probed for either 1 h at room temperature (25°C). or overnight at 4 °C.
Statistical analysis was performed with the SPSS V. 9.0 statistical package (SPSS) and a two-sided unpaired Student's *t*-test for the analysis of differences in mean values between groups.

### Example 1

### NAMPT triggers in vitro myeloid differentiation

To determine whether the G-CSF-dependent increase in NAMPT is of functional consequence, CD34⁺ bone marrow progenitor cells from healthy individuals have been treated with NAMPT alone or with NAMPT in combination with G-CSF using the methods described above. Treatment with NAMPT increased first CD34⁺ cell proliferation and subsequently granulocytic and monocytic differentiation, leading to markedly increased numbers and percentages of common myeloid (CD11b⁺), granulocytic (CD15⁺ and CD16⁺) and monocytic (CD14⁺) cells (Fig. 1a) with the morphology of granulocytes and monocytes (Fig, 1b). Notably, CD16⁺ cell numbers were greater in the NAMPT-treated group than in the G-CSF-treated group, and incubation of CD34⁺ cells with both NAMPT and G-CSF had an additive effect on the number of both CD15⁺ and CD16⁺ cells (Fig. 1a). Moreover, we observed elevated amounts of iNAD⁺ and SIRT1 as well as C/EBPα and C/EBPβ mRNAs in NAMPT-treated cells has been observed compared with the control group (Fig. 2, Fig. 1c,d), which was accompanied by enhanced expression of the C/EBP targets G-CSFR and G-CSF (Fig. 1 e,f).

To determine the effects of intracellular NAMPT on cell differentiation, CD34⁺ cells were transduced with a lentiviral construct containing NAMPT complementary DNA (NAMPT Iv).

**PBEF-1 cDNA synthesis and construction of PBEF-1 cDNA containing lentiviral vector**. The PBEF1 cDNA was PCR amplified, sequenced and subcloned into pGEMT cloning vector. PBEF1 cDNA was excised from pGEMT and cloned into pRRL.PPT.SF.GFPpre using Agel / BsrGI restriction sites to construct pRRL.PPT.SF.PBEF1pre. pRRL.PPT.SF.GFPpre is a derivative of the standard lentiviral vector pRRL.PPT.PGK.GFPpre (kindly provided by Luigi Naldini, Milano, Italy) using an internal SFFV U3 promoter. pRRL.PPT.SF.i2GFPpre incorporates an encephalomyocarditis virus internal ribosomal entry site (IRES2, Clontech) 5' of the GFP sequence. To construct the bicistronic coexpression construct pRRL.PPT.SF.PBEF1.i2GFPpre, the i2GFPpre sequence was cloned into pRRL.PPT.SF.PBEF1pre. HEK 293T cells were used for lentiviral supernatant production. 293T cells were maintained in Dulbeeco's modified Eagles medium (DMEM) supplemented with 10% FCS, 100 U/ml penicillin/streptomycin, and 2 mM glutamine. The day before transfection, 5x10⁶ 293T cells were plated on a 10-cm dish. For transfection, the medium was exchanged and 25 µM chloroquine (Sigma-Aldrich, Munich, Germany) was added. 8 µg transfer vector DNA (pRRL.PPT.SF.PBEF1pre or pRRL.PPT.SF.PBEF1.i2GFPpre) and 5 µg of a VSVg (glycoprotein of the vesicular stomatitis virus) envelope plasmid were used. In addition, 12 µg of a lentiviral gag/pol plasmid (pcDNA3 g/p 4xCTE) and 5 µg of a Rev plasmid (pRSV-Rev, kindly provided by T. Hope, Chicago) was cotransfected using the calcium phosphate technique. Medium was changed after 10-12 h. Transfection efficiency was controlled by FACS analysis. Supernatants containing the viral particles were collected 24-72 h after transfection, filtered through a 0,22-µm filter, concentrated by ultracentrifugation and stored at -80°C until usage.

Lentiviral transduction of CD34⁺ cells. The virus titers for transduction averaged and typically ranged from 1 to 5 x 10⁸ IU/ml after ultracentrifugation. CD34⁺ cells were transduced from three healthy donors with lentiviral supernatants with a multiplicity of infection MOI of 1-2, re-transduced after 24 h and assessed transduction efficiency after 72 h as the percentage of green fluorescent protein (GFP)-positive cells. Transduction with NAMPT Iv led to myeloid differentiation of CD34⁺ cells (Fig. 2b,c) accompanied by upregulation of SIRT1, C/EBPα and C/EBPβ, mRNAs (Fig. 3d). Consistently, treatment with NAMPT or transduction with NAMPT Iv induced *in vitro* myeloid differentiation of CD34⁺ hematopoietic cells from subjects with congenital neutropenia (Fig. 3a-c).

NAMPT-dependent differentiation of the promyelocytic leukemia cell line HL-60 was also evaluated, which does not respond to G-CSF but differentiates into granulocytes upon ATRA treatment. NAMPT induced myeloid differentiation of HL-60 calls, accompanied by reduced cell proliferation, which was similar to the effects of ATRA (Fig. 4a,b). Elevated levels of iNAD⁺ as well as mRNAs of SIRT1, C/EBPα and C/EBPβ in both NAMPT- and ATRA-treated groups were detected, compared with untreated controls (Fig. 4c). Similar results were obtained upon transduction of HL-60 cells with NAMPT Iv. Myeloid differentiation of NAMPT iv-transduced cells was accompanied by increased expression of SiRT1, C/EBPα and C/EBPβ (Fig. 4c,d). These findings indicate that NAMPT acts similarly to ATRA in the hierarchy of molecular effectors operating during neutrophil differentiation in this G-CSF-unresponsive promyelocytic cell line.

### Example 2

### Nicotinamide promotes in vitro granulopoiesis

NAMPT is needed to generate NAD⁺ from nicotinamide. Accordingly, the effects of nicotinamide on *in vitro* myelopoiesis were analyzed (Fig. 5). Similarly to NAMPT, nicotinamide induced myeloid differentiation of CD34⁺ cells in a dose-dependent
manner, as assessed by FACS analysis (Fig. 5a). Moreover, stimulation of CD34⁺ cells with nicotinamide resulted in increased levels of C/EBPα and C/EBPα mRNAs, as well as of G-CSFR mRNA and protein expression (Fig. 5b,d). G-CSF protein amounts in the culture medium of nicotinamide-treated CD34⁺ cells were also significantly higher as compared to untreated cells (Fig. 5c).

### Example 3

### NAMPT is essential for G.CSF-dependent granulopolesis

NAMPT is induced by G-CSF, so it was further analyzed whether this enzyme is required for G-CSF-triggered granulopoiesis. CD34⁺ cells were pretreated with FK866 (National institute of Mental Health, Chemical Synthesis and Drug Supply Program, Dr. Ken Rehder), a specific inhibitor of NAMPT, and subsequently stimulated with G-CSF. Treatment with 1 nM FK866 had no effect on cell viability and proliferation, but abrogated G-CSF-triggered granulocytic differentiation (Fig. 6a), Moreover, when NAMPT activity was inhibited G-CSF was unable to induce C/EBPα or C/EBPβ mRNA expression (Fig. 6b), nor did it induce G-CSFR mRNA or protein expression (Fig. 6c), Further, no increase in G-CSF or iNAD⁺ levels in G-CSF- or NAMPT-stimulated cells pretreated with FK866 were detected.

Notably, inhibition of G-CSFR by transduction of CD34+ cells with G-CSFR-specific short hairpin RNA (shRNA) abolished the effects of NAMPT on myeloid differentiation and on mRNA expression of myeloid-specffic factors, although GMCSF-induced differentiation remained unaffected (Fig. 6d).

### G-CSFR shRNA synthesis, construction of G-CSFR shRNA expression cassette and shRNA containing lentiviral vectors

DNA oligonucleotides corresponding to position 642 bp to 660 bp of the human CSF3R gene sequence were chemically synthesized. These nucleotides also contained overhang sequences from a 5' *Bgl*II- and a 3'*Hind*III- restriction sites. The numbering of the first nucleotide of the shRNAs refers to the ATG start codon. The oligonucleotide sequences was as follows GCTTCACTCTGAAGAGTTT (Seq. ID No. 3). The oligonucleotides were inserted into the *Bgl*II/*Hind*III-digested pSUPER-derived plasmid to generate pSUPER-CSF3R_642 and verified the isolated clone by DNA sequencing. To generate lentiviral transgenic plasmids containing shRNA expression cassettes located in the U3 region of the □3'-LTR pRRL,PPT.SF.DsRedEx.pre was used. pRRL.PPT.SF.DsRedEx.pre is a derivative of the standard lentiviral vector pRRL.PPT.PGK, DsRedEx.pre (kindly provided by Luigi Naldini, Milano, Italy) using an internal SFFV promoter. To generate the lentiviral CSF3R_642-SR plasmid, the pSUPER-CSF3R_642 was digested with *Sma*I and *Hinc*II and ligated the resulting DNA fragments (360 bp) into the *Sna*BI site of the pRRL.PPT.SF.DsRedEx,pre. The lentiviral plasmid encodes RFP_{EXPRESS} as reporter gene.
This finding supports a previously undescribed role and specificity for NAMPT and NAD⁺ in the G-CSF-G-CSFR signaling pathway.

### Example 4

### Vitamin 83 induces neutrophilic granulopoiesis in vivo

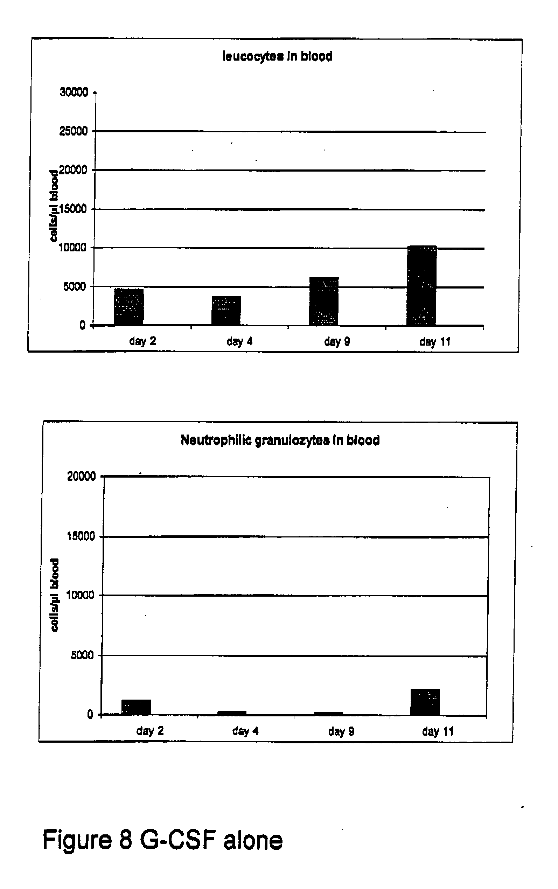

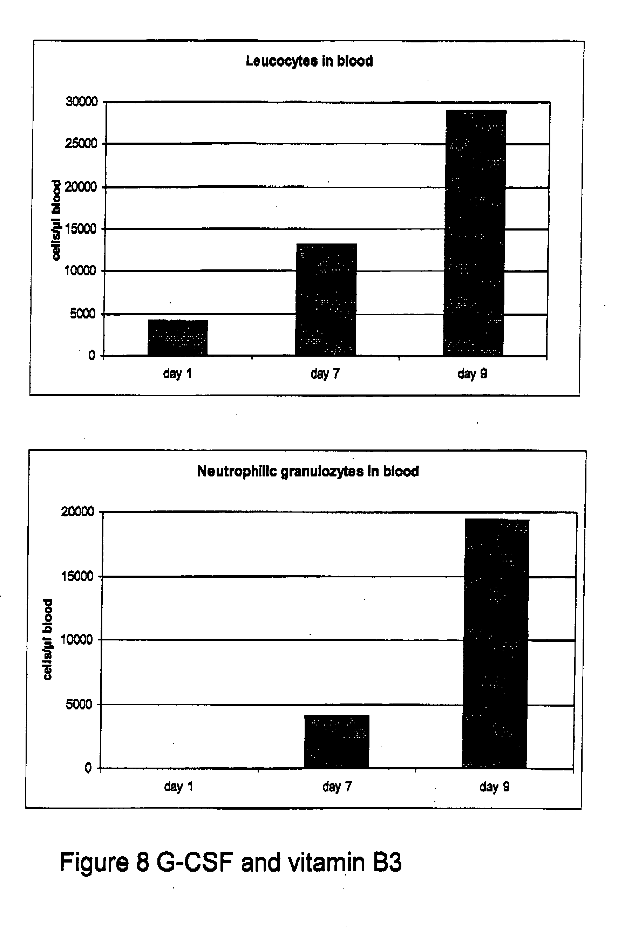

Based on the *in vitro* data showing induction of granulocytic differentiation by NAMPT or its substrate nicotinamide, it was investigated whether treatment of healthy individuals with vitamin B3 had similar effects on granulopoiesis. Indeed, treatment of six healthy individuals with vitamin B3 (10 mg per kg per d) for 1 week resulted in significant increases in the numbers of neutrophilic granulocytes in all subjects (Fig, 7a), Increased neutrophil numbers were detected early (days 1-2) after the initiation of vitamin B3 treatment, with a decline to the physiological level after 2-3 d of vitamin B3 discontinuation (Fig. 7a). Increased iNAD⁺ levels in PMNs from vitamin B3-treated individuals, compared with their steady-state levels were observed (Fig. 7b). Additionally, it was found that vitamin B3 induces upregulation of C/EBPα and C/EBPβ, mRNA levels in bone marrow CD34⁺ cells, CD33⁺ cells and in peripheral blood PMNs, as well as elevation of G-CSFR mRNA and protein expression in the PMNs (Fig. 7c,d, Fig. 8a,b). Other blood cell lineages were unaffected, and no adverse effects of vitamin B3 were detected in the treated individuals. Consistent with the initial observation of positive effects of NAMPT on *in vitro* neutrophil differentiation of human CD34⁺ cells, the results collectively indicate that the NAMPT substrate nicotinamide can increase neutrophil numbers in peripheral blood when administered as an oral drug.

### Example 5

One patient suffering from severe congenital neutropenia was treated with 2,5 µg/kg/day of G-CSF alone or in combination of Vitamin B3 (20 mg/kg/day) and G-CSF (2,5 Mg/kg/day). The number of leukocytes and absolute number of neutrophilic granulocytes were measured at the indicated time points (Fig 8).

### DISCUSSION

Individuals with congenital neutropenia present a valuable clinical model to study myeloid differentiation. In these individuals neutrophil granulopoiesis is 'arrested' at the promyelocytic stage, and the G-CSF response of myeloid progenitors is abrogated *in vitro* and *in vivo.* Therefore, determination of mechanisms of G-CSF-induced myeloid differentiation in subjects with congenital neutropenia may facilitate the delineation of new G-CSF signaling pathways. On the basis of this clinical model, an essential role of the nicotinamide-NAMPT-NAD⁺ pathway in G-CSF-triggered myelopoiesis has been shown. It is the first report describing a connection between G-CSF-activated signaling, which triggers granulocytic differentiation, and the metabolic processes regulating cellular functions via NAD⁺. NAMPT is a key biosynthetic enzyme converting nicotinamide to nicotinamide adenine mononucleotide, the main source of NAD⁺. Increased amounts of NAMPT in mammalian cells cause upregulation of NAD⁺, which supports the hypothesis that this enzyme is the rate-limiting determinant for NAD⁺ production. Recent studies show that NAMPT is both an extracellular and an intracellular enzyme. However, it remained unclear whether NAMPT is enzymatically active in the bloodstream and whether its other biological effects are attributable to its role in producing NAD⁺. Increased amounts of NAMPT and its product NAD⁺ have been dedicated in plasma and in myeloid cells from G-CSF-treated healthy individuals and even higher concentrations of both molecules In subjects with congenital neutropenia. Moreover, it has been documented herein that treatment or transduction with NAMPT induces myeloid differentiation of hematopoietic cells from healthy individuals and subjects with congenital neutropenia. This finding supports that NAMPT is able to generate NAD⁺ from both extracellular and intracellular nicotinamide and that the biological effects of NAMPT are indeed tightly linked to downstream NAD⁺ synthesis. Extracellularly generated NAD⁺ thus appears able to enter the cell and contribute to the intracellular activities of NAD⁺, No cellular receptor for NAMPT or alternative mechanism for its intracellular uptake has been clearly identified to date.
The data herein show that high intracellular NAMPT and subsequently elevated NAD⁺ levels are essential for G-CSF-triggered myeloid differentiation of CD34⁺ hematopoietic progenitor cells *in vitro.* This observation is supported by the finding that suppression of NAMPT with the specific inhibitor FK866 substantially abrogated G-CSF-induced myeloid differentiation. Moreover, treatment of CD34⁺ hematopoietic progenitors with NAMPT alone induced granulocytic differentiation via upregulation of G-CSF and G-CSFR.
It is assumed postulate that NAMPT induces a G-CSF autoregulatory loop via the following sequential mechanism. First, G-CSF promotes increased extra- and intracellular NAMPT expression. Second, high levels of NAMPT lead to increased NAD⁺ production and upregulation of SIRT1. Third, SIRT1 interacts with and activates the granulocyte-specific transcription factors C/EBPα and C/EBPβ. Last, these transcription factors bind the promoters of the genes encoding G-CSF and G-CSFR and activate their expression, leading to a positive feedback regulation of G-CSF and NAMPT synthesis and activity.

In myeloid progenitor cells from individuals with congenital neutropenia, pharmacological doses of G-CSF markedly increased NAMPT, NAD⁺ and SIRT1 levels, Recently, we reported that in these cells from patients with congenital neutropenia the granulocyte-specifc transcription factors LEF-1 and C/EBPα are severely downregulated, WO 2008/034637 and thus C/EBPα would not be available for binding to SIRT1. C/EBPα and C/EBPβ have different functions in granulopoiesis. C/EBPα is responsible for steady-state granulopoiesis, whereas C/EBPβ is crucial for cytokine-induced 'emergency' granulopoiesis, In contrast to C/EBPα, normal or even elevated amounts of C/EBPβ are expressed in myeloid progenitor cells from individuals with congenital neutropenia. It is assumed that high NAMPT, NAD⁺ and SIRT1 levels activate a C/EBPβ-dependent emergency pathway of neutrophil granulopoiesis in individuals with congenital neutropenia, whereas the steady-state regulation by LEF-1 and C/EBPα does not function. These results support the hypothesis that in individuals with congenital neutropenia, treatment with pharmacological doses of G-CSF leads to activation of a NAMPT-dependent autoregulatory loop and emergency granulopoiesis via C/EBP-β. It was additionally confirmed the importance of NAMPT for myeloid differentiation, on the basis of the finding that treatment of the myeloid leukemia cell line HL-60 with NAMPT resulted in differentiation toward granulocytes.

Further, an *in vitro* and *in vivo* effect of vitamin B3 (nicotinamide) on neutrophilic granulocyte differentiation in healthy individuals was observed. Vitamin B3 is biosynthetically converted to NAD⁺ by NAMPT. The recommended daily dose of vitamin B3 is 0.3 mg kg⁻¹ (20 mg for an adult), but recent clinical studies in subjects with type 1 diabetes reveal no side effects of high nicotinamide doses (25-50 mg kg⁻¹ d⁻¹). Herein, application of 10-20 mg kg⁻¹ d⁻¹ of vitamin B3 led to a marked increase in the neutrophilic granulocyte content (up to twofold within 2 d) without affecting any other blood cell lineages.

It is concluded that G-CSF stimulates NAMPT expression, with subsequent upregulation of NAD⁺ and SIRT1 synthesis in myeloid progenitors and neutrophils from subjects with congenital neutropenia. NAMPT and its product NAD⁺ are essential factors in steady-state and stress-induced (for example, infection-induced) emergency neutrophil granulopoiesis. Moreover, the present findings present new perspectives in clinical and experimental medicine, supporting the therapeutic application of vitamin B3 for the treatment of clinical syndromes associated with cytopenia like chronic or acute neutropenia.

## Claims

1. Nicotinic acid, nicotinamide, nicotinamide adenine mononucleotide, nicotinamide adenine dinucleotide or derivatives thereof as active ingredients for preventing or treating all types of cytopenia of the myeloid or lymphoid linage.

2. NAMPT or a functional fragment or homolog thereof, or an enhancer or an inducer of NAMPT expression, NAMPT activity or NAMPT-mediated signalling as an active ingredient for preventing or treating all types of cytopenia of the myeloid or lymphoid lineage.

3. Nicotinic acid, nicotinamide, nicotinamide adenine mononucleotide, nicotinamide adenine dinucleotide or derivatives thereof or NAMPT or a functional fragment or homolog thereof according to claim 1, or an enhancer or an inducer of NAMPT expression, NAMPT activity or NAMPT-mediated signalling according to claim 2 as active ingredient for supporting chemotherapy.

4. Nicotinic acid, nicotinamide, nicotinamide adenine mononucleotide, nicotinamid adenine dinucleotide or derivatives thereof according to claim 1, or NAMPT or a functional fragment or homolog thereof, or an enhancer or an inducer of NAMPT expression, NAMPT activity or NAMPT-mediated signalling of claim 2 as active ingredients for treating febrile neutropenia.

5. Nicotinic acid, nicotinamide, nicotinamide adenine mononucleotide, nicotinamid adenine dinucleotide or derivatives thereof of claim 1, or NAMPT or a functional fragment or homolog thereof, or an enhancer or an inducer of NAMPT expression, NAMPT activity or NAMPT-mediated signalling of claim 2 as active ingredients for preventing or treating cytopenia of the myeloid lineage.

6. Nicotinic acid, nicotinamide, nicotinamide adenine mononucleotide, nicotinamid adenine dinucleotide or derivatives thereof of claim 1, or NAMPT or a functional fragment or homolog thereof, or an enhancer or an inducer of NAMPT expression, NAMPT activity or NAMPT-mediated signalling of claim 2, as active ingredients for preventing or treating neutropenia.

7. Nicotinic acid, nicotinamide, nicotinamide adenine mononucleotide, nicotinamid adenine dinucleotide or derivatives thereof according to claim 1, or NAMPT or a functional fragment or homolog thereof, or an enhancer or an inducer of NAMPT expression, NAMPT activity or NAMPT-mediated signalling of claim 2 as active ingredients for treating severe congenital neutropenia.

8. Nicotinic acid, nicotinamide, nicotinamide adenine mononucleotide, nicotinamid adenine dinucleotide or derivatives thereof according to claim 1 or NAMPT or a functional fragment or homolog thereof, or an enhancer or an inducer of NAMPT expression, NAMPT activity or NAMPT-mediated signalling of claim 2 as active ingredients for mobilising and/or inducing differentiation of stem cells *in vivo* or *in vitro.*

9. A pharmaceutical composition comprising as active ingredients a therapeutically active amount of nicotinic acid, nicotinamide, nicotinamide adenine mononucleotide, nicotinamid adenine dinucleotide or derivatives thereof and a therapeutically active amount of G-CSF.

10. A pharmaceutical composition comprising as active ingredients a therapeutically effective amount of NAMPT or a functional fragment or homolog thereof, or an enhancer or an inducer of NAMPT expression, NAMPT activity or NAMPT-mediated signalling and a therapeutically active amount of G-CSF.

11. The pharmaceutical composition according to claim 8 for preventing or treating all types of cytopenia of the myeloid or lymphoid lineage or for preventing or treating different types of neutropenia or for mobilising of hematopoietic stem cells and/or inducing differentiation of stem cells *in vivo* or *in vitro.*

12. NAMPT or a functional fragment or homolog thereof, or an enhancer or an inducer of NAMPT expression, activity or NAMPT-mediated signalling according to anyone of claims 2 to 11 wherein the active ingredient is provided in the form of a protein or a nucleic acid sequence.

13. An inhibitor of NAMPT expression NAMPT activity and/or NAMPT-mediated signaling for preventing or treating leukaemia.

14. An inhibitor of NAMPT expression NAMPT activity and/or NAMPT-mediated signalling or antagonist of NAMPT according to claim 13 for treating or preventing ALL CML or AML in particular, cancer of the myeloid lineage with transformed granulocytes.

15. The inhibitor of NAMPT expression, NAMPT activity and/or NAMPT signalling according to 13 or 14 wherein the inhibitor of NAMPT is siRNA or shRNA, miRNA, antisense nucleic acid molecules or RNAi, small molecules or peptides acting as NAMPT specific inhibitors.
